# EUROPEAN PATENT APPLICATION

(11) **EP 2 481 724 A1**
(43) Date of publication of application: **01.08.2012**
(21) Application number: 12164420.7
(22) Date of filing: 06.08.2008
(51) Int. Cl.: C07C 265/12, C07D 401/04, C07F 3/02

(54) **Method for producing hydrazine compound, and production intermediates of hydrazine compound and methods for producing the intermediates**

(30) Priority: 14.08.2007 JP 2007211325; 14.08.2007 JP 2007211326
(62) Divisional of application: 08827189.5
(71) Applicant: Sumitomo Chemical Company, Limited, Tokyo 104-8260 (JP)
(72) Inventor: The designation of the inventor has not yet been filed
(74) Representative: Vossius & Partner

(57) **Abstract**

Provided are a novel method for producing a hydrazine compound useful as an insecticidal compound, and production intermediates which can be suitably used for the production method and methods for producing the intermediates. As the production intermediates, a specific isocyanate compound and an organomagnesium compound represented by the following general formula (IV) are provided. The organomagnesium compound can be synthesized from a corresponding dibromo compound.

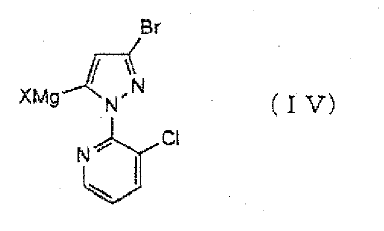

## Description

### TECHNICAL FIELD

The present invention relates to a method for producing a hydrazine compound which is useful as an active ingredient of an insecticide and so on, and production intermediates which are suitably used for the method and methods for producing the intermediates.

### BACKGROUND ART

The hydrazine compound represented by the following general formula (I) is known to be useful as an insecticidal compound (International Publication WO 2007-043677 pamphlet (Patent Document 1)).

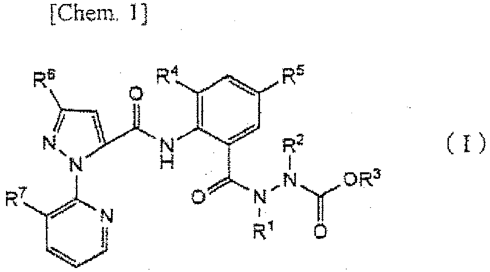

In the formula, R¹ and R² each represent an alkyl group having 1 to 6 carbons; R³ represents an alkyl group having 1 to 6 carbons, an alkoxyalk-yl group having 3 to 6 carbons, an alkenyl group having 3 to 6 carbons or an alkynyl group having 3 to 6 carbons; R⁴ represents halogen, an alkyl group having I to 6 carbons or a perfluoroalky group having 1 to 6 carbons; and R⁵ represents hydrogen, halogen, a cyano group, an alkyl group having 1 to 6 carbons or a perfluoroallcyl group having I to 6 carbons; R⁶ represents hydrogen, halogen, a cyano group, an alkyl group having I to 6 carbons optionally substituted with halogen, an alkoxy group having I to 6 carbons optionally substituted with halogen, an alkylthio group having 1 to 6 carbons optionally substituted with halogen, an alkylsulfinyl group having 1 to 6 carbons optionally substituted with halogen or an alkylsulfonyl having 1 to 6 carbons optionally substituted with halogen; and R⁷ represents halogen or an alkyl group having 1 to 6 carbons optionally substituted with halogen.

The document describes, as a method for producing the hydrazine compound represented by the general formula (I), a production method in which carboxylic acid represented by the following general formula (B) is derived from an organometallic compound represented by the following general formula (A) and the carboxylic acid is used as a production intermediate.

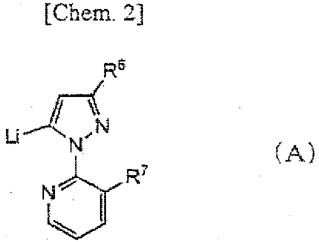

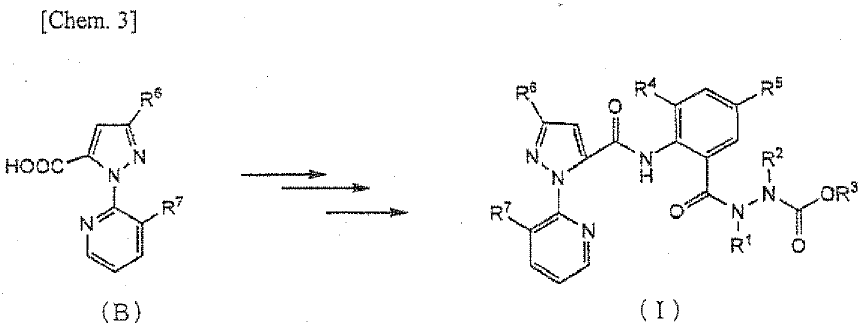

In the formulae, R¹ to R⁷ are the same as described above.
[Patent Document 1] International Publication WO 2007-043677 pamphlet

### DISCLOSURE OF THE INVENTION

### PROBLEMS TO BE SOLVED BY THE INVENTION

An object of the present invention is to provide a novel method which can produce the insecticidal compound represented by the general formula (I) more efficiently from the organometallic compound, which generally requires careful handling, represented by the general formula (A). Another object of the present invention is to provide production intermediates which can be suitably used for the production method and methods for producing the intermediates.

### MEANS FOR SOLVING THE PROBLEMS

The present invention provides a method for producing a hydrazine compound, including a step of
reacting an isocyanate compound represented by the following general formula (II): 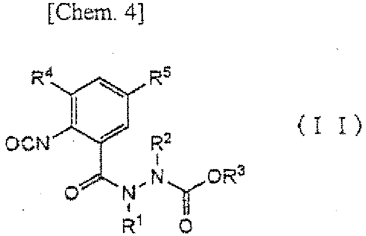 wherein R¹ and R² each independently represent an alkyl group having 1 to 6 carbons; R³ represents an alkyl group having 1 to 6 carbons, an alkoxyalkyl group having 3 to 6 carbons, an alkenyl group having 3 to 6 carbons or an alkynyl group having 3 to 6 carbons; R⁴ represents halogen, an alkyl group having 1 to 6 carbons or a perfluoroalkyl group having I to 6 carbons; and R⁵ represents hydrogen, halogen, a cyano group, an alkyl group having 1 to 6 carbons or a perfluoroalkyl group having 1to 6 carbons,
with an organometallic compound represented by the following general formula (III): 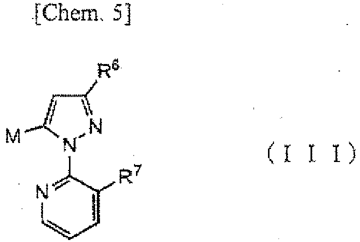 wherein R⁶ represents hydrogen, halogen, a cyano group, an alkyl group having 1 to 6 carbons optionally substituted with halogen, an alkoxy group having 1 to 6 carbons optionally substituted with halogen, an akylthio group having 1 to 6 carbons optionally substituted with halogen, an alkylsulfinyl group having I to 6 carbons optionally substituted with halogen or an alkylsulfonyl group having I to 6 carbons optionally substituted with halogen; R⁷ represents halogen or an alkyl group having I to 6 carbons optionally substituted with halogen; M represents Li, MgX or ZnX; and X represents chlorine, bromine or iodine,
the hydrazine compound being represented by the following general formula (I): 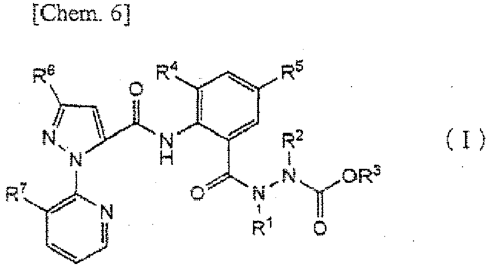 wherein R¹ to R⁷ are the same as described above. It is preferred that M in the general formula (III) is MgX.

An isocyanate compound represented by the following general formula (II): 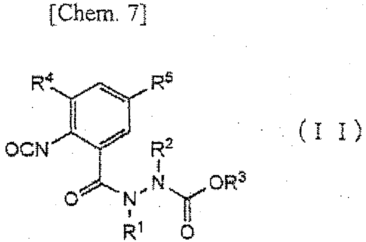 wherein R¹ and R² each independently represent an alkyl group having I to 6 carbons; R³ represents an alkyl group having 1 to 6 carbons, an alkoxyalkyl group having 3 to 6 carbons, an alkynyl group having 3 to 6 carbons or an alkynyl group having 3 to 6 carbons; R⁴ represents halogen, an alkyl group having 1 to 6 carbons or a perfluoroalkyl group having 1 to 6 carbons; and R⁵ represents hydrogen, halogen, a cyano group, an alkyl group having 1 to 6 carbons or a perfluoroalkyl roup having I to 6 carbons,
is also provided by the present invention.

The present invention also provides an organomagnesium compound represented by the following general formula (IV): 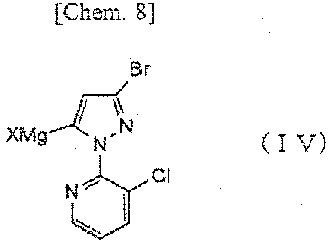 wherein X represents chlorine, bromine or iodine. The organomagnesium compound is a compound within the scope of the compound represented by the general formula (III),

The present invention also provides a dibromo compound represented by the following formula (V): 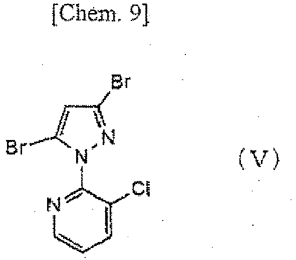 and a method for producing the organomagnesium compound represented by the general formula (IV), including a step of reacting the dibromo compound, with a Grignard compound represented by the following general formula (VI):

R-MgX (VI)

wherein R represents an alkyl group having 1 to 6 carbons or a vinyl group; and X represents chlorine, bromine or iodine.

A method for producing a formyl compound, including a step of reacting the organomagnesium compound represented by the general formula (IV) with a compound represented by the following general formula (VII):

HC(=O)-Q (VII)

wherein Q represents an amino group substituted with two alkyl groups each having 1 to 6 carbons or an alkoxy group having 1 to 3 carbons,
the formyl compound being represented by the following formula (VIII): 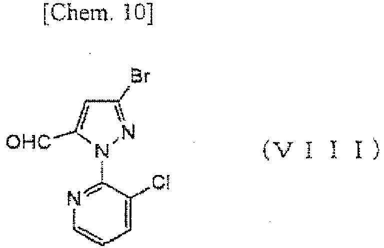 is further provided by the present invention,

### EFFECTS OF THE INVENTION

According to the present invention, there are provided a novel method for producing the hydrazine compound represented by the general formula (I) which employs an isocyanate compound and an organometallic compound as production intermediates, and production intermediates suitably usable for the production method.

The present invention also provides the organomagnesium compound represented by the general formula (IV) which is useful as a production intermediates usable for the above-described method and its producing method. The organomagnesium Compound is more stable than a corresponding organolithium compound and use of the compound enables derivation of other production intermediates which are useful in the production of an insecticidal compound such as the hydrazine compound represented by the general formula (I). Such stability can reduce burdens in terms of industrial production facility.

### BEST MODES FOR CARRYING OUT THE INVENTION

### <Production Method of Hydrazine Compound>

The hydrazine compound according to the present invention is a compound represented by the following general, formula (I): 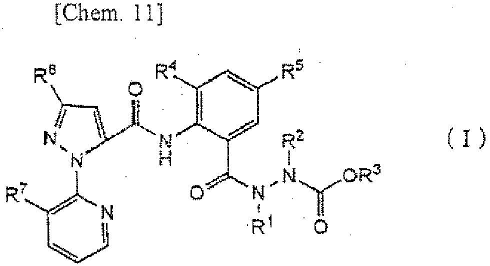 and is useful as an active ingredient of an insecticide and so on. In the present invention, the hydrazine compound is produced by reacting an isocyanate compound represented by the following general formula (II): 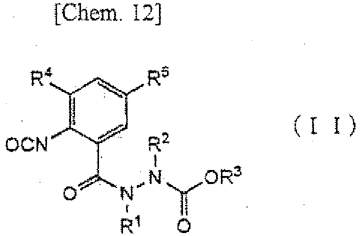 with an organometallic compound represented by the following general formula (III):

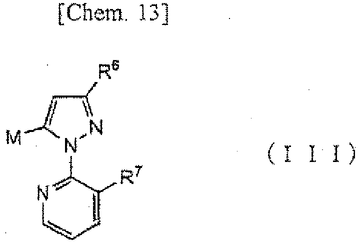

In the general formulae (I) and (II), R¹ and R² each independently represent an alkyl group having 1 to 6 carbons, R₃ represents an alkyl group having 1 to 6 carbons, an alkoxyalkyl group having 3 to 6 carbons, an alkenyl group having 3 to 6 carbons or an alkynyl group having 3 to 6 carbons, R⁴ represents halogen, an alkyl group having I to 6 carbons or a perfluoroalkyl group having 1 to 6 carbons, and R⁵ represents hydrogen, halogen, a cyano group, an alkyl group having I to 6 carbons or a perfluoroalkyl group having I to 6 carbons.

As for R¹ and R². examples of the alkyl group having I to 6 carbons include a methyl group, an ethyl group, a propyl group, an isopropyl group, a butyl group, an isobutyl group, a sec-butyl group, a. pentyl group and a hexyl group. As for R³, examples of the alkyl group having I to 6 carbons include a methyl group, an ethyl group, a propyl group, an isopropyl group, a butyl group, an isobutyl group, a sec-butyl group, a pentyl group and a hexyl group; examples of the alkoxyalkyl group having 3 to 6 carbons include a 2-methoxyethyl group, a 2-ethoxyethyl group and a 2-isopropyloxyethyl group; examples of the alkenyl group having 3 to 6 carbons include a. 2-propenyl group, a 2-butenyl group, a 3-butenyl group, a. 2-methyl-2-propenyl group, a 3 -methyl-2-butenyl group, a 2-pentenyl group and a 2-hexenyl group; and examples of the alkynyl group having 3 to 6 carbons include a 2-propynyl group, a 2-butynyl group and a 3-butynyl group. As for R⁴ and R⁵, examples of halogen include F, Cl, Br and I; examples of the alkyl group having 1 to 6 carbons include a methyl group, an ethyl group, a propel group, an isopropyl group, a butyl group, an isobutyl group, a see-butyl group, a pentyl group and a hexyl group; and examples of the perfluoroalkyl group having I to 6 carbons include a trifluoromethyl group and a pentafluoroethyl group.

In the general formulae (I) and (III), R⁶ represents hydrogen, halogen, a cyano group, an alkyl group having 1. to 6 carbons optionally substituted with halogen, an alkoxy group having I to 6 carbons optionally substituted with halogen, an alkylthio group having I to 6 carbons optionally substituted with halogen, an alkylsulfinyl group having I to 6 carbons optionally substituted with halogen or an alkylsulfonyl group having I to 6 carbons optionally substituted with halogen, and R⁷ represents halogen or an alkyl group having I to 6 carbons optionally substituted with halogen.

Specific examples of the hydrazine compound represented by the general formula (I) include the following compounds:
(a) a compound wherein R¹ is a methyl group, R² is a methyl group, R³ is a methyl group, R⁴ is a methyl group, R⁵ is Cl, R⁶ is Br, and R⁷ is Cl;
(b) a compound wherein R¹ is a methyl group, R² is a methyl group, R³ is a methyl group, R⁴ is a methyl group, R⁵ is a cyano group, R⁶ is Br, and R⁷ is Cl;
(c) a compound wherein R¹ is a methyl group, R². is a methyl group, R³ is a methyl group, R⁴ is Br, R⁵ is Br, R⁶ is Br, and R⁷ is Cl;
(d) a compound wherein R¹ is a methyl group, R² is a methyl group, R³ is a methyl group, R⁴ is Cl, R⁵ is Cl, R⁶ is Br, and R⁷ is Cl;
(e) a compound wherein R¹ is a methyl, group, R² is a methyl group, R³ is a methyl group, R⁴ is a methyl group, R⁵ is Cl, R⁶ is a trifluoromethyl group, and R⁷ is Cl;
(f) a compound wherein R¹ is a methyl group, R² is a methyl group, R³ is a methyl group, R⁴ is a methyl group, R⁵ is Br, R⁶ is a trifluoromethyl group, and R⁷ is Cl;
(g) a compound wherein R¹ is a methyl group, R² is a methyl group, R³ is a methyl group, R⁴ is a methyl group, R⁵ is hydrogen, R⁶ is a trifluoromethyl group, and R⁷ is Cl; and
(h) a compound wherein R¹ is a methyl group, R² is a methyl group, R³ is a methyl group, R⁴ is Br, R⁵ is Cl, R⁶ is a trifluoromethyl group, and R⁷ is Cl.

In the general formula. (III), M represents a metal-containing group and specifically represents Li, MgX or ZnX. X represents chlorine, bromine or iodine. In consideration of the stability and yield of the organometallic compound of the general formula (III), the yield of the hydrazine compound represented by the general formula (I) and the like, M is preferably MgX and more preferably MgCl or MgBr. As the compound represented by the general formula (III) and in which M is MgX, for example, the organomagnesium compound represented by the general formula (IV) to be described later can be suitably used.

As long as they have the substituents R¹ to R⁷defined above, each of the compounds represented by the general formulae (I) to (III) may be, a mixture of two or more compounds which are different in the kind of the substituents.

In the reaction of the isocyanate compound represented by the general formula (II) with the organometallic compound represented by the general formula (III), the amount of the organometallic compound to be used is usually about 0.8 to 10 mol and preferably about 1.0 to 2.0 mol per mol of the isocyanate compound.

The reaction temperature is usually about -80 to 1OO°C and preferably about -10 to 30°C. The reaction time is usually about 0,1 to 100 hours and typically about 1 to 10 hours, although it depends on the reaction temperature, an amount and a kind of the organometallic compound of the general formula (III) to be used, and so on.

The reaction is usually performed in the presence of a solvent. Examples of the solvent include aromatic hydrocarbons such as toluene and xylene, aliphatic hydrocarbons such as hexane, heptane and cyclohexane, ethers such as dioxane, tetrahydrofuran, t-butylmethylether, 1,2-di-n-butoxyethane and diethyleneglycol di-n-butylether, and a mixed solvent thereof The solvent is preferably tetrahydrofuran or a mixed solvent of tetrahydrofuran and an aromatic hydrocarbon. The amount of the solvent to be used can be set at about 1 to 100 parts by mass and preferably about 1 to 30 parts by mass per part by mass of the isocyanate compound represented by the general formula (II).

More specifically, the reaction of the isocyanate compound with the organometallic compound can be performed by adding, to a, solution S1 containing the isocyanate compound, a solution S2 containing the organometallic compound. The solutions S1 and the S2 may be the isocyanate compound itself the reaction mixture upon preparation and the organometallic compound itself, respectively. The solution S1 containing the isocyanate compound may be a solution prepared by isolating the isocyanate compound from the reaction mixture and then dissolving the isolated isocyanate compound in an appropriate solvent. The solvent in the solution S1 and the solvent in the solution S2 may be the same or different from each other.

The hydrazine compound contained in the reaction mixture after completion of the reaction is preferably isolated and is used, as an insecticidal compound, for pesticides. The hydrazine compound can be isolated by subjecting the reaction mixture to usual post-treatment such as washing, phase-separation and concentration, for example. The isolated hydrazine compound may be used after further purification by recrystallization, column chromatography or the like.

### <Production Method of Isocyanate Compound>

The method for producing the isocyanate compound represented by the general formula (II) is not particularly limited; however, suitably, a method of isocyanating an amino group of a corresponding amino compound (an aniline derivative) represented by the following general formula (IX): 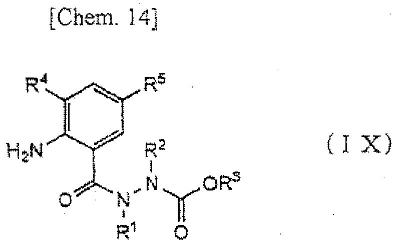 with an isocyanating agent is applied. In the formula, R¹ to R⁵ are the same as in the case of the general formula (II). Examples of the isocyanating agents include known ones, such as triphosgene (bis(trichloromethyl)carbonate), diphosgene (trichloromethyl chlorofonnate), and phosgene. When using triphosgene as the isocyanating agent, the using amount can be set at about 0.3 to 3.3 mol and preferably about 0.33 to 0.66 mol per mol of the amino compound. When using diphosgene as the isocyanating agent, the using amount can be set at about 0.455 to 5 mol and preferably about 0.5 to 1..0 mol per mol of the amino compound. When using phosgene as the isocyanating agent, the using amount can be set at about 0.8 to 10 mol and preferably about 1.0 to 2.0 mol per mol of the amino compound. Examples of a reaction solvent include aromatic hydrocarbons such as toluene and xylene, halogenated aromatic hydrocarbons such as chlorobenzene, aliphatic hydrocarbons such as hexane, heptane and cyclohexane, halogenated aliphatic hydrocarbons such as 1,2-dichloroethane and chloroform, ethers such as dioxane and tetrahydrofuran, esters such as ethyl acetate and butyl acetate, ketones such as methyl isobutyl ketone and methyl ethyl ketone, and a. mixed solvent thereof. Other reaction conditions can be those conditions that can be usually employed by those skilled in the art in isocyanation of an amino compound.

After completion of the reaction, the isocyanate compound may be isolated by usual post-treatment such as concentration and distillation to, separate the solvent, the excess isocyanating agent and hydrogen chloride as a byproduct, or alternatively, the mixture containing the isocyanate compound may be subjected to the reaction with an organometallic compound in the next step without isolation of the isocyanate compound by omitting a part or all of the post-treatment.

The amino compound represented by the general formula (IX) can be prepared by the following method, for example.

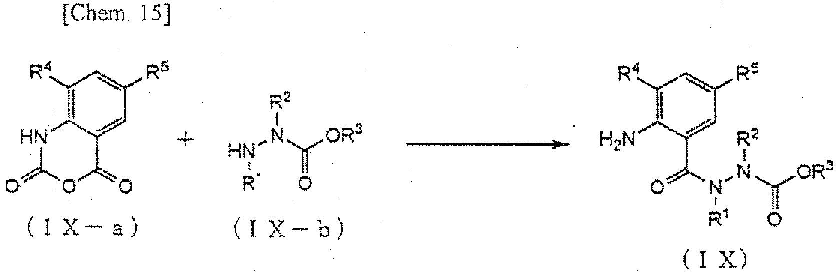

The compound represented by the general formula (IX-a) may be a known compound or can be prepared by a. known method in which a corresponding 2-aminobenzoic acid derivative react with phosgenes. The compound represented by the general formula (IX-b) may be a known compound or can be prepared by a known method in which corresponding hydrazines react with a chloroformate,

### <Production Method of Organometallic Compound>

Among the organometallic compounds represented by the general formula (III), an organomagnesium compound wherein M is MgX (X represents chlorine, bromine or iodine) can be prepared by, for example, reacting a corresponding bromo compound represented by the following general formula (X): 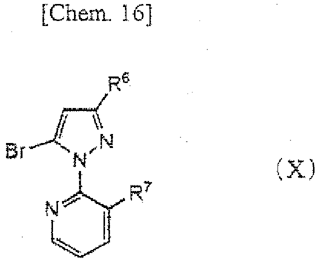 with a Grignard compound represented by the following general formula (VI):

R-MgX (VI).

In the general formula (X), R⁶ and R⁷ are the same as in the case of the general formula. (III). X in the general formula (VI) represents chlorine, bromine or iodine, and preferably chlorine or bromine. The Grignard compound represented by the general formula (VI) may be a mixture of two or more compounds which are different from one another in X.

R. in the general formula (VI) represents an alkyl group hazing 1 to 6 carbons or a vinyl group, and preferably represents an alkyl group having 1 to 3 carbons from the viewpoint of the yield of the organomagnesium compound to be obtained, and so on. Specific examples of the alkyl group having 1 to 6 carbons include a methyl group, an ethyl group, a propyl group, an isopropyl group, a butyl group, an isobutyl group and a hexyl group,

The amount of the Grignard compound of the general formula (VI) to be used in the production of the organomagnesium compound using the Grignard compound represented by the general formula (VI) is usually about 0,5 to 5 mol, preferably about 1 to 3 mol per mol of the bromo compound represented by the general formula (X).

The reaction temperature is usually about -80 to 100°C and preferably in the range of about -20 to 30°C. The reaction time is usually about 0.1 to 100 hours and typically about 1 to 24 hours, although it depends on the reaction temperature, an amount and a kind of the Grignard compound of the general formula (VI) to be used, and so on.

The reaction using the Grignard compound is preferably performed in the presence of a solvent. Examples of the solvent include aromatic hydrocarbons such as toluene and xylene, aliphatic hydrocarbons such as cyclohexane, ethers such as dioxane and tetrahydrofuran, and a mixed solvent thereof. The solvent is preferably tetrahydrofuran or a mixed solvent of tetrahydrofuran and an aromatic hydrocarbon. The amount of the solvent to be used can be set at, for example, about 0.1 to 100 parts by mass and preferably about I to 20 parts by mass per part by mass of the bromo compound represented by the formula (X), In the present invention, the reaction using the Grignard compound is preferably performed by dropping a solution S4 containing the Grignard compound represented by the general formula (VI) into a solution S3 containing the bromo compound represented by the formula (X), In this case, the amount of the solvent is the total amount of the solvents contained in the solutions S3 and S4, The solvent in the solution S3 and the solvent in the solution S4 may be the same or different from each other. The Grignard compound represented by the general formula (VI) can be prepared by a conventionally known method.

The progress of the reaction can be checked by taking out a part of the reaction mixture and quenching the mixture with an appropriate reagent, and thereafter qualitatively or quantitatively analyzing the reaction product of the reagent and the organomagnesium compound as well as the bromo compound as a raw material using an analytical means such as chromatography, e.g. thin-layer chromatography, gas chromatography or high-performance liquid chromatography, or NMR The reagent for quenching is not particularly limited as long as the organomagnesium compound is converted into a compound which can be detected by the above-described means, and example thereof include water, deuterium oxide, alcohols, carbon dioxide, chloroformates, N,N-dimethylformamidehereinafter, may be referred to as DMF), dimethyl sulfate, methyl chloride and acetone. When, for example, deuterium oxide is used as the reagent for quenching, the -MgX group of the organomagnesium compound is replaced by a -D group to give a compound represented by the following formula (XII): 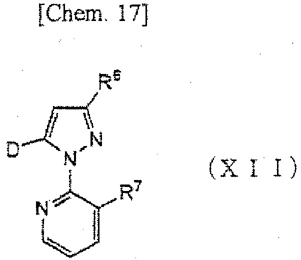 which can be quantitatively analyzed by chromatography. The organomagnesium compound can be converted into a carboxylated product, an alkoxycarbonylated product, a formylated product, a methylated product and so on depending on the kind of the reagent for quenching.

In a preferred aspect, the organomagnesium compound contained in the reaction mixture is not isolated and the reaction mixture itself is subjected to the reaction with the above-described isocyanate compound. The organomagnesium compound represented by the general formula (III) and in which M is MgX has better stability compared with a corresponding organolithium compound. Accordingly, storage of the reaction mixture and the reaction using the reaction mixture do not necessarily have to be performed under a very low temperature of-80 to -70°C.

Among the organometallic compounds represented by the general formula (III), an organolithium compound wherein M is Li can be prepared by, for example, reacting a corresponding compound represented by the following general formula (XI): 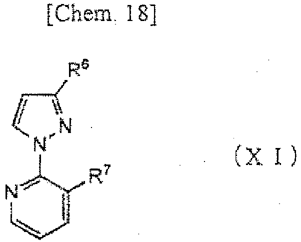 with lithium diisopropylamide (hereinafter, may be referred to as LDA).

The organometallic compound represented by the general formula (III) and in which M is ZnX (X represents chlorine, bromine or iodine) can be prepared by reacting a compound wherein Mis Li or MgX with a zinc salt.

The bromo compound represented by the general formula (X) can be prepared by the following method, for example. The following example shows a case where R⁶ is Br and R⁷ is Cl. Details of the preparation method will be described later.

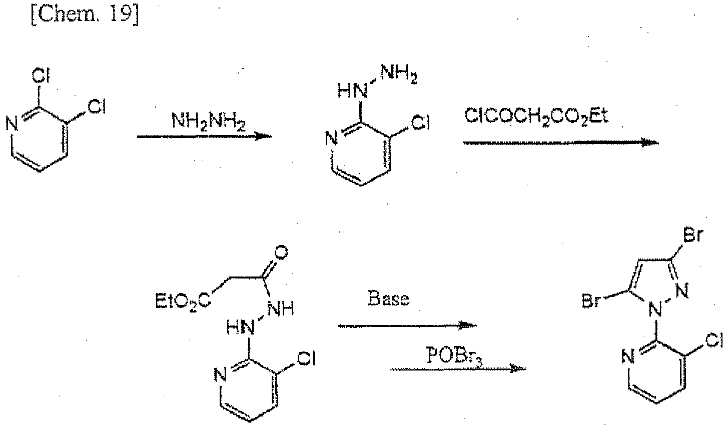

The compound represented by the general formula (XI) can be prepared by the following method, for example.

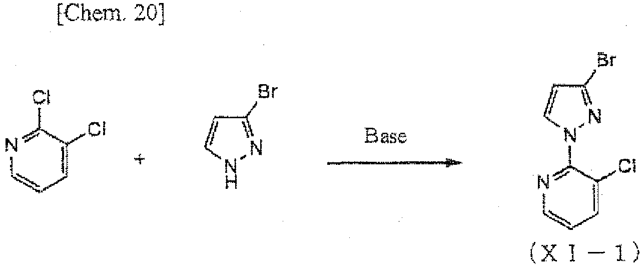

### <Organomagnesium Compound>

Next, the organomagnesium compound represented by the following general formula (IV): 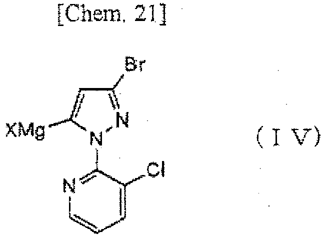 which is provided by the present invention is specifically described. The compound is more stable than a corresponding organolithium compound and is extremely useful as a production intermediate of an insecticidal compound represented by the general formula (I) or the following general formula (I'). The organomagnesium compound of the present invention has a -MgX group on its pyrazole ring and is a kind of the so-called Grignard reagent as well as a compound within the scope, of the compound represented by the above-described general formula (III).

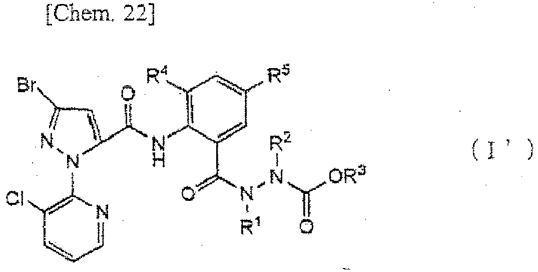

In the general formula (I'), R¹ represents a C1-C6 alkyl group optionally substituted with halogen, R² represents hydrogen or a C1-C6 alkyl group optionally substituted with halogen, R³ represents a C1-C6 alkyl group optionally substituted with halogen, a C3-C6 alkoxyalkyl group optionally substituted with halogen, a C3-C6 alkenyl group optionally substituted with halogen or a C3-C6 alkynyl group optionally substituted with halogen, R⁴ represents halogen or a C1-C6 alkyl group optionally substituted with halogen, and R⁵ represents hydrogen, halogen, a cyano group or a C1-C6 alkyl group optionally substituted with halogen.

In the general formula (IV), X represents halogen and specifically, chlorine, bromine, iodine and so on. Preferably, X is chlorine or bromine. The organomagnesium compound of the present invention may be a mixture of two or more organomagnesium compounds which are different from one another in X. The organomagnesium compound of the present invention has higher stability in storage than a lithium salt represented by the following formula (C). That is, the lithium salt represented by the following formula (C) is not stable under a low temperature condition of about -10°C and may require a very low temperature condition of, for example, - 78°C for the storage. Such storage under a very low temperature condition may be burdensome in terms of facility requirements particularly in industrial production. Meanwhile, the organomagnesium compound represented by the general formula (IV) has higher stability in storage and more suitable for industrial production

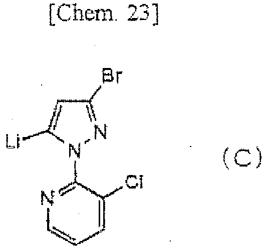

As a method for producing the organomagnesium compound of the present invention represented by the general formula (IV), the following method can be suitably used. That is, the method is a method in which a dibromo compound represented by the following formula (V): 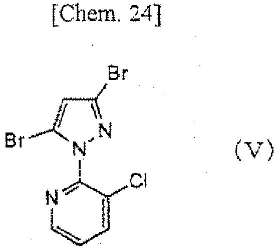 is reacted with a Grignard compound represented by the following general formula (VI):

R-MgX (VI).

X in the general formula (VI) is as described above. From the viewpoint of the yield of the organomagnesium compound to be obtained, and so on, X preferably represents chlorine or bromine. The Grignard compound represented by the general formula (VI) may be a. mixture of two or more compounds which are different from one another in X. R represents an alkyl group having 1 to 6 carbons or a vinyl group, and preferably represents an alkyl group having 1 to 6 carbons from the viewpoint of the yield of the organomagnesium compound to be obtained, and so on. Specific examples of the alkyl group having 1 to 6 carbons include a methyl group, an ethyl group, a propyl group, an isopropyl group, a butyl group, an isobutyl group and a hexyl group

By the reaction using the Grignard compound of the general formula (VI), the organomagnesium compound of the present invention can be obtained at high yield. In addition, in the reaction using the Grignard compound of the general formula (VI), an insertion reaction of Mg into Br at the 3 -position of the pyrazole ring scarcely occurs and it is possible to selectively obtain the organomagnesium compound of the present invention.

The amount of use of the Grignard compound of the general formula (VI) in the production of the organomagnesium compound of the present invention using the Grignard compound represented by the general formula (VI) is usually about 0.5 to 5 mol, preferably about 1 to 3 mol per mol of the dibromo compound represented by the formula (V).

The reaction temperature is usually about -80 to 100°C and preferably in the range of about -20 to 30°C. The reaction time is usually about 0.1 to 100 hours and typically about 0.1 to 3 hours, although it depends on the reaction temperature, an amount and a kind of the Grignard compound of the general formula (VI) to be used, and so on.

The reaction using the Grignard compound is usually performed in the presence of a solvent. Examples of the solvent include aromatic hydrocarbons such as toluene and xylene, aliphatic hydrocarbons such as cyclohexane, ethers such as dioxane and tetrahydrofuran, and a mixed solvent thereof The solvent is preferably tetrahydrofuran or a mixed solvent of tetrahydrofuran and an aromatic hydrocarbon The amount of the solvent to be used can be set at, for example, about 0.1 to 100 parts by mass and preferably about 3 to 20 parts by mass per part by mass of the dibromo compound represented by the formula (V). In the present invention, the reaction using the Grignard compound is preferably performed by dropping a solution S6 containing the Grignard compound represented by the general formula (VI) into a solution S5 containing the dibromo compound represented by the formula (V). In this case, the amount of the solvent is the total amount of the solvents contained in the solutions S5 and S6. The solvent in the solution S5 and the solvent in the solution S6 may be the same or different from each other. The Grignard compound represented by the general formula (VI) can be prepared by a conventionally known method.

The progress of the reaction can be checked by taking out a part of the reaction mixture and quenching the mixture with an appropriate reagent, and thereafter qualitatively or quantitatively analyzing the reaction product of the reagent and the organomagnesium compound as well as the dibromo compound as a raw material using an analytical means such as chromatography, e.g. thin-layer chromatography, gas chromatography or high-performance liquid chromatography, or NMR The reagent for quenching is not particularly limited as long as the organomagnesium compound is converted into a compound which can be detected by the above-described means, and examples thereof include water, deuterium oxide, alcohols, carbon dioxide, chloroformates, DMF, dimethyl sulfate, methyl chloride and acetone. When, for example, deuterium oxide is used as the reagent for quenching, the -MgX group of the organomagnesium compound is replaced by a -D group to give a compound represented by the following formula (XII'): 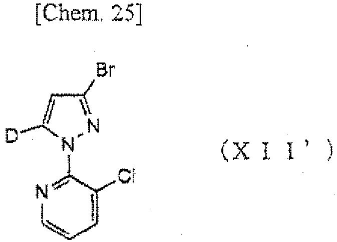 which can be quantitatively analyzed by chromatography. The organomagnesium compound can be converted into a carboxylated product, an alkoxycarbonylated product, a formylated product, a methylated product and so on depending on the kind of the reagent for quenching. As the reagent for quenching, specific formyl compounds to be described later may be used. Provision of these conversion products by quenching demonstrates that the reaction with the Grignard compound generates the organomagnesium compound represented by the general formula (IV).

In a preferred aspect, the organomagnesium compound contained in the reaction mixture after completion of the reaction may be subjected to the reaction with the isocyanate compound represented by the general formula (II) without isolation for the production of the insecticidal compound represented by the general formula (I) or (I'), or may be converted into another production intermediate. The organomagnesium compound of the present invention has good stability and accordingly, storage of the reaction mixture and the reaction using the reaction mixture do not necessarily have to be performed under a very low temperature of-80 to -70°C. Examples of the other production intermediates include carboxylated product in which the -MgX group of the organomagnesium compound is substituted with a carboxyl group and a formylated product in which the group is sub stituted with a formyl group, The carboxylated product can be obtained by, for example, bringing the reaction mixture into contact with carbon dioxide gas. The formylated product in which the -MgX group has been converted into a formyl group, that is, a compound represented by the following formula (VIII): 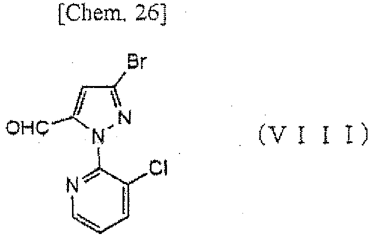 can be obtained by reacting the organomagnesium compound with a. compound represented by the following general formula (VII):

HC(=O)-Q (VII).

In the general formula (VII), Q represents an amino group substituted with two alkyl groups each having 1 to 6 carbons or an alkoxy group having 1 to 3 carbons. The amino group is preferably substituted with two alkyl groups and in this case, each alkyl group is more preferably an alkyl group having 1 to 3 carbons. Specific examples of the compound represented by the general formula. (VII:) include formamides such as N,N-dimethylformamide and N,N-diethylformamide and formates such as methyl formate, ethyl formate, n-propyl formate and isopropyl formate. Preferably, formamides can be mentioned.

The reaction with the compound represented by the general formula (VII) can be specifically performed by mixing the organomagnesium compound with the compound represented by the general formula (VII). The reaction can also be performed by adding the compound represented by the general formula (VII) to the reaction mixture containing the organomagnesium compound. The amount of use of the compound represented by the general formula (VII) is usually about 0.8 to 10 mol and preferably about 1.0 to 5 mol per mol of the organomagnesium compound represented by the formula (IV).

The reaction temperature is usually about -80 to 100°C and preferably in the range of about -20 to 30°C The reaction time is usually about 0.1 to 100 hours and typically about 0.1 to 5 hours.

After completion of the reaction, the formylated product can be isolated by performing usual post-treatment operations such as washing, phase-separation and concentration. The isolated formulated product can be further purified by recrystallization, column chromatography and so on. Alternatively, the isolated formylated product can be used for the next step without being purified. Further alternatively, it is also possible to proceed to the next step without performing a part or all of the post-treatment operations.

### <Dibromo Compound>

The dibromo compound represented by the formula (V) can be suitably produced by the following method using industrially available raw materials.

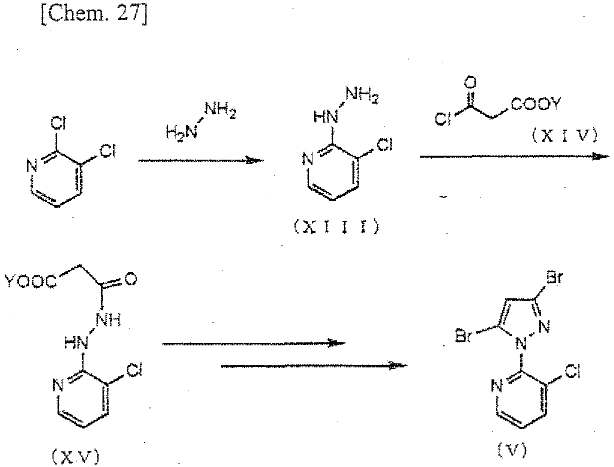

In the following, each steps will be described.

First, 2,3-dichloropyridine is reacted with hydrazine to give a hydrazine compound (XIII). In this reaction, the amount of use of hydrazine is usually about I to 10 mol and preferably about 2 to S mol per mol of 2,3-dichloropyridine. The reaction temperature is usually about 50 to 100°C. The reaction time is usually about 10 to 100 hours. Examples of a solvent that can be used include alcohols such as n-butanol, aromatic hydrocarbons such as toluene and xylene, halogenated aromatic hydrocarbons such as chlorobenzene, aliphatic hydrocarbons such as hexane and heptane, alicyclic hydrocarbons such as cyclohexane, halogenated aliphatic hydrocarbons such as chloroform, ethers such as dioxane and tetrahydrofuran, water, and a mixed solvent thereof

Then, the hydrazine compound (XIII) is reacted with a carboxylic acid chloride (XIV) to give an ester compound (XV) The hydrazine compound (XIII) is 3-chloropyridine-2-ylhydrazine and the carboxylic acid chloride (XTV) is an alkoxycarbonyl group-containing carboxylic acid chloride. Y in the carboxylic acid chloride (XIV) represents an alkyl group having 1 to 3 carbons. Specific examples of the carboxylic acid chloride (XIV) include methyl malonyl chloride and ethyl malonyl chloride. Preferably, ethyl malonyl chloride is used. The ester compound (XV) obtained by the reaction may be in the form of a hydrochloride salt.

In this reaction, the amount of use of the carboxylic acid chloride (XIV) is usually about 1 to 10 mol and preferably about I to 5 mol per mol of the hydrazine compound (XIII). The reaction temperature is usually about -10 to 100°C and preferably in the range of about 0 to 30°C. The reaction time is usually about 0.1 to 10 hours. Examples of a solvent include aromatic hydrocarbons such as toluene and xylene, halogenated aromatic hydrocarbons such as chlorobenzene, aliphatic hydrocarbons such as hexane and heptane, alicyclic hydrocarbons such as cyclohexane, halogenated aliphatic hydrocarbons such as chloroform, ethers such as dioxane and tetrahydrofuran, esters such as ethyl acetate, ketones such as methyl isobutyl ketone, alkyl nitriles such as acetonitrile and propionitrile, and a mixed solvent thereof Among these, acetonitrile is particularly preferably used.

After completion of the reaction, usual post-treatment operations may be performed to isolate the ester compound (XV) or a part or all of the post-treatment operations may be omitted to proceed the reaction to the next step.

The next step is to obtain a dibromo compound (V) from the ester compound (XV) or the hydrochloride salt thereof. The step consists of a first operation of reacting the ester compound (XV) with a base and a second operation of reacting the compound obtained by the first operation with a brominating agent. Examples of the base usable in the first operation include metal hydroxides such as sodium hydroxide, potassium hydroxide and lithium hydroxide. The amount of the base to be used is about 1 to 10 mol per mol of the ester compound (XV).

The reaction temperature can be set at about -10 to 100°C and preferably in the range of about 0 to 50°C. The reaction time is usually about 0.1 to 100 hours and typically about 1 to 10 hours. Examples of a solvent include alcohols such as methanol, ethanol and n-butanol, aromatic hydrocarbons such as toluene and xylene, halogenated aromatic hydrocarbons such as chlorobenzene, aliphatic hydrocarbons such as cyclohexane, halogenated aliphatic hydrocarbons such as chloroform, ethers such as dioxane and tetrahydrofuran, esters such as ethyl acetate, ketones such as methyl isobutyl ketone, alkyl nitriles such as acetonitrile and propionitrile, aprotic highly polar solvents such as DMF, N-methylpyrrolidone (hereinafter, may be abbreviated as NMP), 1,3-dimethylimidazolidinone (hereinafter, may be abbreviated as DMI) and dimethylsulfoxide (hereinafter, may be abbreviated as DMSO), water and a mixed solvent thereof. Among these, alcohols such as methanol, ethanol and n-butanol are particularly preferably used. After completion of the reaction, the reaction mixture is neutralized and post-treatment operations such as extraction with an organic solvent and concentration are performed to isolate an intermediate product.

Examples of the brominating agent usable in the subsequent second operation include phosphorus oxybromide and phosphorus pentabromide. The amount of use of the brominating agent in the case of phosphorus oxybromide is about 2 to 10 mol and preferably about 2 to 5 mol per mol of the ester compound (XV). Phosphorus oxybromide may also be used as the reaction solvent. The reaction temperature can be set at about 0 to 200°C and preferably in the range of about 30 to 120°C. The reaction time is usually about 1 to 100 hours and typically about 5 to 20 hours. When using a solvent, examples of the solvent include halogenated aliphatic hydrocarbons such as chloroform, alkyl nitriles such as acetonitrile and propionitrile, polyphosphoric acid, and a mixed solvent thereof

After completion of the reaction, the dibromo compound represented by the formula (V) can be isolated by performing usual post-treatment operations. The isolated dibromo compound may be purified by recrystallization, column chromatography and so on before preparation of the above-described organomagnesium compound. Alternatively, the dibromo compound may be used for the preparation of the organomagnesium compound without performing a part or all of the post-treatment operations and purification.

In the following, the present invention will be described more specifically with reference to examples, but the present invention is not limited to these examples.

### [Preparation of Organometallic Compound]

### <Example 1>

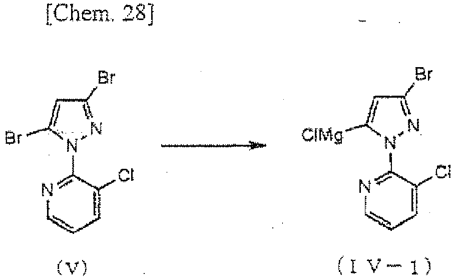

In 1.71g of anhydrous THF, 0.171 g of a dibromo compound (V) was dissolved. Then, 0.96 g of a solution of isopropylmagnesium chloride (i-PrMgC1) (11% in THF, ca. 1 mol/L) was added dropwise thereto at room temperature and the mixture was kept at the same temperature for 15 minutes to prepare a solution containing an organomagnesium compound (IV-1).

### <Example 2>

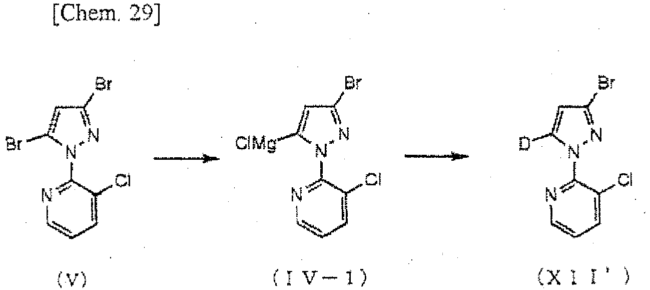

In 3.40 g of anhydrous THF, 0.3376 g of the dibromo compound (V) was dissolved and the solution was cooled to -10°C. Then, a solution of isopropylmagnesium chloride (i-PrMgC1) (11% in THF, 2.0 equivalent relative to the dibromo compound (V)) was added dropwise thereto at -10°C and the mixture was kept at the same temperature to generate the organomagnesium compound (IV-1) in the reaction system. After 30 minutes of heat retention, 0.2 mL of the reaction mixture solution was sampled and quenched with deuterium oxide. The product after quenching was measured by using ¹H-NMR, whereby it was confirmed that the dibromo compound (V) did not remain and had almost entirely been converted into .a deuterated product (XII').

### <Reference Example 1>

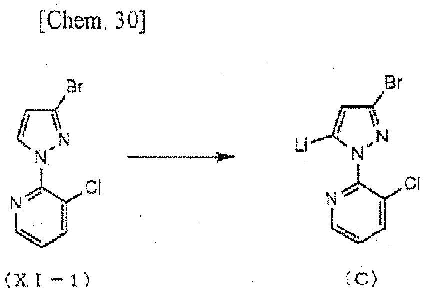

A mixture of 5.0 g of a compound (XI-1) and 30 mL of tetrahydrofuran was cooled to -78°C. Thereafter, to the mixture was added dropwise 11.7 mL of a 2.0 M solution of lithium diisopropylamide in heptane/tetrahydrofuran/ethylbenzene so that a solution containing a compound (C) was prepared.

### <Reference Example 2>

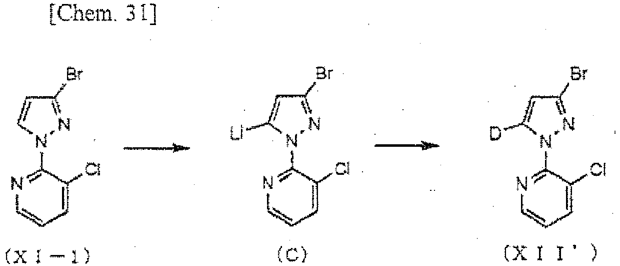

In about 10 g of anhydrous THF, 1.03 g of the compound (XI-1) was dissolved and the solution was cooled to -10°C. Then, a solution of lithium diisopropylamide (i-Pr₂NLi) in THF (1.1 equivalent relative to the compound (XT-1)) was added dropwise thereto at -10°C and the mixture was kept at the same temperature. After 30 minutes of heat retention, 0.2 mL of the reaction mixture solution was sampled and quenched with deuterium oxide. The product after quenching was measured by using ¹H-NMR, whereby it was confirmed that 34% of the compound (XI-1) had been converted into the deuterated product (XII'), In addition, a by-product almost in the same amount with the compound (XII") was confirmed.

### [Preparation of Formyl Compound (VIII)]

### <Examples 3>

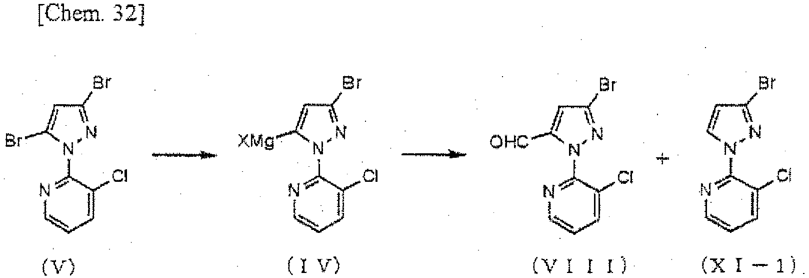

In 3,40 g of anhydrous THF, 0.3376 g of the dibromo compound (V) was dissolved and the solution was cooled to -80°C Then, 1.87 g of a solution of isopropylmagnesium chloride (i-PrMgCl) (11% in THF, ca. 1 mol/L) was added dropwise thereto at - 80 to -75°C and the mixture was kept at the same temperature to generate organomagnesium compound (IV) in the reaction system After 30 minutes of heat retention, 0.2 mL of the reaction mixture solution was sampled and quenched with deuterium oxide. The product after quenching was measured by using ¹H-NMR, whereby it was confirmed that 96.8% of the dibromo compound (V) had been converted into the deuterated product (XII'). After a total of 1 hour of heat retention, 0-11 g of ethyl formate was added to the reaction mixture solution at the same temperature and then kept at -80 to -75°C for 1.5 hours, at -50 to -45°C for 1 hour, at -10 to -9°C for 1 hour, and at room temperature for 14 hours. To the resulting mixture was added 4.04 g of water at room temperature, and it was extracted with 3.37 g of ethyl acetate and then made phase-separation. The organic layer was sequentially washed with 3-37 g of water and 3.37 g of saturated brine, dried over magnesium sulfate, and concentrated to give a crude product of a formylated product (VIII). The crude product was subjected to ¹H-NMR measurement, whereby it was confirmed that the dibromo compound (V) did not remain and the crude product was composed of 10% of the formylated product (VIII) and 90% of the compound (XI-1).

### <Example 4>

In 3.38 g of anhydrous THF, 0.3373 g of the dibromo compound (V) was dissolved and the solution was cooled to -10°C. Then, 1.87 g of a solution of isopropylmagnesium chloride (11% in THF, ca. 1 mol/L) was added dropwise thereto at -11 to -10°C and the mixture was kept at -11 to -9°C for 30 minutes to generate the organomagnesium compound (IV) in the reaction system. At that time, the reaction mixture solution was sampled and quenched with deuterium oxide. The product after quenching was measured by using ¹H-NMR, whereby it was confirmed that 100% of the dibromo compound (V) had been converted into the deuterated product (XII'). The product was further warmed up to room temperature and kept at the same temperature for 35 minutes. Thereafter, the reaction mixture solution was again cooled to -12 to - 9°C. At the same temperature, 0.22 g of ethyl formate was added thereto and the mixture was kept at -12 to -10°C for 33 minutes. Thereafter, the mixture was further added with 0.23 g of ethyl formate at the same temperature and kept at 0 to 5°C for 30 minutes. At the same temperature, 0.67 g of water was added thereto to quench the mixture, and 3.37 g of water was added to the resulting mixture at room temperature and extracted with 3.37 g of ethyl acetate and then made phase-separation. The organic layer was sequentially washed with 3.37 g of water and 3.37 g of saturated brine, dried over magnesium sulfate, and concentrated to give a crude product of the formylated product (VIII). The crude product was subjected to ¹H-NMR measurement, whereby it was confirmed that the dibromo compound (V) did not remain and the crude product was composed of 23% of the formylated product (VIII) and 77% of the compound (XI-1).

### <Example 5>

In 3.39 g of anhydrous THE, 0.3371 g of the dibromo compound (V) was dissolved and the solution was cooled to -10°C. Then, 1.88 g of a solution of isopropylmagnesium chloride (11% in THF, ca. 1 mol/L) was added dropwise thereto at -13 to -8°C and the mixture was kept at -17 to -4°C for 38 minutes to generate the organomagnesium compound (IV) in the reaction system. Thereafter, 0.23 g of DMF was added thereto at -12 to -8°C and the mixture was kept at -12 to -9°C for 1 hour. Then, 1.69 g of a saturated aqueous ammonium chloride solution was added thereto at the same temperature and the mixture was further warmed up and kept at room temperature at 20°C for 3 hours and left standing still at room temperature overnight. To the resulting mixture was added 3.37 g of water, and it was extracted with 3.37 g of ethyl acetate and then made phase-separation. The organic layer was sequentially washed with 3.37 g of water and 3.37 g of saturated brine, dried over magnesium sulfate, and concentrated to give a crude product of the formylated product (VIII). The crude product was subjected to ¹H-NMR measurement, whereby it was confirmed that the dibromo compound (V) did not remain and the crude product was composed of 50% of the formylated product (VIII) and 50% of the compound (XI-I).

### <Examples 6>

In 3.39 g of anhydrous THF, 0.3373 g of the dibromo compound (V) was dissolved. Then, 1,89 g of a solution of isopropylmagnesium chloride (11% in THE, ca. 1 mol/L) was added dropwise thereto at 8 to 24°C and the mixture was stirred at 20 to 26°C for 2 hours to generate the organomagnesium compound (IV) in the reaction system. Thereafter, 0.23 g of DMF was added thereto at 25 to 28°C and the mixture was kept at 25 to 28°C for 2.5 hours. Then, 1.70 g of a saturated aqueous ammonium chloride solution was added thereto at the same temperature. To the resulting mixture which was cooled to 10°C was added 3,37 g of water, and it was extracted with 3.36 g of ethyl acetate and then made phase-separation. The organic layer was sequentially washed with 3.39 g of water and 3.37 g of saturated brine, dried over magnesium sulfate, and concentrated to give 0.254 g of the formylated product (VIII) (yield: 87,5%). The resulting formylated product (VIII) was subjected to ¹H-NMR measurement, whereby it was confirmed that the dibromo compound (V) did not remain and 100% of the product was the formylated product (VIII).

The ¹H-NMR data of the obtained formylated product (VIII) are as follows. ¹H-NNM(CDCl₃, TMS)δ(ppm);7.11(s, 1H), 7.47(dd, 1H), 7.96(d, 1H), 8.54(d, 1H), 9.79(s, 1H).

### [Preparation of 3-chloro-2-(3,5-dibromopyrazole-1-yl)pyridine (dibromo compound (V))]

### <Examples 7>

### (1) Preparation of 3-cmoropyridine-2-ylhydrazine (hydrazine compound (XIII))

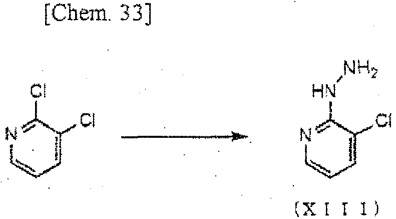

To a mixture of 200.88 g of 2,3-dichloropyridine and 402.10 g of n-butanol, 203.83 g of hydrazine monohydrate and 183.85 g of potassium carbonate were added at room temperature. Thereafter, the mixture was heated under stirring and kept being stirred for 31 hours at an internal temperature of 109 to 111°C, The resulting reaction mixture was cooled to 29°C, added with 401.0 g of water, stirred for 3D minutes at room temperature and then filtrated, The residue on the filter was sequentially washed with 100 g of n-butanol and 200 g of water, and dried under vacuum at 50°C so that 187.81 g of 3-chloropyridine-2-ylhydrazine (hydrazine compound (XIII)) was obtained.

The ¹H-NMR data of the obtained 3-chloropyridine-2-ylhydrazine are as follows. ¹H-NMR(CDCl₃, TMS)δ(ppm):8.10(dd, 1H), 7.47(dd, 1H), 6.65(dd, 1H), 6.24(bs, 1H), 3.98(bs, 2H).

### (2) Preparation of ethyl [N'-(3-chloropyridine-2-yl)-hydrazmocarbonyl]acetate (ester compound (XV-1))

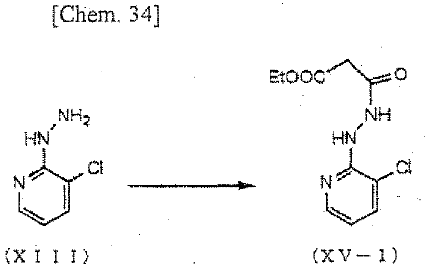

To a mixture of 14.25 g of 3-chloropyridine-2-ylhydrazine (hydrazine compound (XIII)) and 142.50 g of acetonitrile, 15.00 g of ethyl malonyl chloride with a content of 90% was added dropwise with stirring at an internal temperature in the range of 21 to 25°C. The resulting mixture was kept being stirred for 2 hours at the same temperature, 1.00 g of ethyl malonyl chloride was added thereto, and kept being stirred at room temperature overnight. The resulting reaction mixture was concentrated under reduced pressure and dried under vacuum at 40°C to give 29,64 g of ethyl [N'-(3-chloropyridine-2-yl)-hydrazinocarbonyl]acetate hydrochloride (ester compound (XV-1)).

The ¹H-NMR data of the obtained ethyl [N'-(3-chloropyridine-2-yl)-hydrazinocarbonyl]acetate hydrochloride are as follows.
¹H-NMR(DMSO-d₆, TMS)δ(ppm):10.53(bs, 1H), 8.07(dd, 1H), 8.01(d, 1H), 6,95(dd, 1H), 4.10(q, 2H), 3.46(s, 2H), 1.21(t, 3H).

### (3) Preparation of 3-chloro-2-(3,5-dibromopyrazole-1-yl)pyridine (dibromo compound (V))

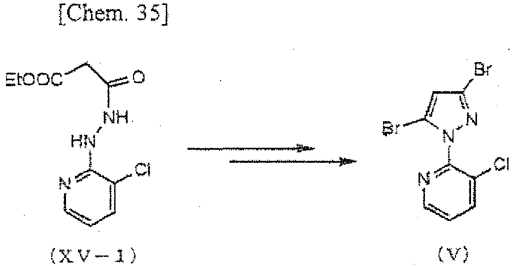

A mixture of 29,00 g of ethyl [N'-(3-chloropyridine-2-yl)-hydrazinocarbonyl]acetate hydrochloride (ester compound (XV-1)) and 290.00 g of 99.5% ethanol was cooled. While stirring the mixture, 300 mL of a solution of caustic soda with a concentration of 1 mol/L in. 99.5% ethanal was added dropwise thereto at an internal temperature of 4 to 10°C. After keeping stirring the mixture for 3 hours at room temperature, the internal temperature was 24.6°C. The resulting mixture was cooled again, and 20 mL of a solution of caustic soda with a concentration of 1 mol/L in 99.5% methanol was added thereto dropwise at an internal temperature of 10°C or lower. Thereafter, the mixture was kept being stirred at room temperature overnight. The resulting reaction mixture was cooled and the pH of the reaction solution was adjusted to 3.97 by adding cone hydrochloric acid dropwise at an internal temperature of 20°C or lower. Thereafter, the solvent and water were removed from the mixture under reduced pressure and the residue was dried under vacuum at 40°C, so that 46.77 g of a powder was obtained. To the powder was added 140.31 g of water and the mixture was stirred at room temperature for 1 hour and filtrated. The residue on the filter was poured with 140.31 g of water for washing and dried under reduced pressure, so that 19.67 g of an intermediate product was obtained.

A mixture of 9.00 g of the intermediate product obtained by the above-described operation and 25.23 g of phosphorus oxybromide was heated and stirred at 100°C for 14 hours. The resulting reaction mixture was cooled to room temperature and then 50 g of water and 50 g of monochlorobenzene (MCB) were added thereto and stirred. Then, to the mixture was added dropwise 21.14 g of a 48% aqueous caustic soda solution at an interval temperature of 6 to 13°C while being cooled to adjust the pH to 10.39. Then, the solid matter was removed by filtration and water and MCB were added to the filtrate and then made phase-separation. The organic layer was concentrated to give 4.55 g of a residue. The residue was purified by silica gel chromatography to give 3.21 g of 3-chloro-2-(3,5-dibromopyrazole-1-yl)pyridine (dibromo compound (V)). The ¹H-NMR measurement was performed, whereby it was confirmed that the purity was 100%. The area percentage (HPLC area percent purity) in high-performance liquid chromatography was 92.4%.

The ¹H-NMR data of the obtained 3-chloro-2-(3,5-dibrornopyrazole-1-yl)pyridine are as follows.
¹H-NMR(CDCl₃, TMS)δ(ppm):8.55(d, 1H), 7.95(d, 1H), 7.7(dd, 1H), 6.53(s, 1H).

### [Synthesis of Amino Compound (IX)]

### <Reference Example 3>

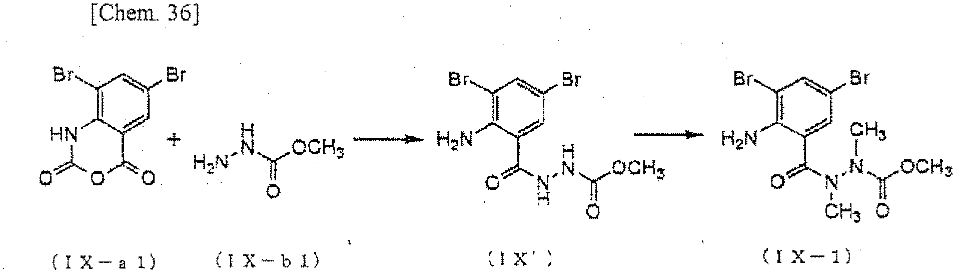

To a mixture of 1.85 g of methyl carbazate (compound (IX-b1)) and 60 mL of tetrahydrofuran, 6,0 g of 6,8-dibromo-2H-3,1-benzoxazine-2,4-1H-dione (compound (IX-al), a compound described in Joual of Organic Chemistry (1947), 12, 743-51) was added under ice cooling, and the mixture was stirred for 3 hours under ice cooling. To the reaction mixture heated to room temperature was further added 0.46 g of methyl carbazate and the mixture was stirred at room temperature for 15 hours. The reaction mixture was concentrated under reduced pressure, and water was poured into the resulting residue. The remaining solid was separated by filtration. The solid was sequentially washed with water and ethyl acetate, so that 4.96 g of N-(2-aruino-3,5-dibromobenzoyl)-N'-methoxycarbonylhydrazine (compound (IX')) was obtained.

The ¹H-NMR data of the compound (IX') are as follows.
¹H-NMR(DMSO-d₆, TMS)δ(ppm):3.63(s, 3H), 6.55(s, 2H), 7.71(s, 1H), 7.79(s, 1H). 9.25(s, 1H), 10.32(s, 1H).

Then, to a mixture of 3.67 g of the compound (IX'), 3.04 g of potassium carbonate and 50 mL of N-methylpyrrolidone was added dropwise a mixture of 3.12 g of methyl iodide and 2 mL of 1-methyl-2-pyrrolidinone under ice cooling and the mixture was stirred for 4 hours under ice cooling and further stirred for 3 hours at room temperature. Water was poured into the reaction mixture and extracted with ethyl acetate. The organic layer was washed with water, dried over anhydrous magnesium sulfate, and concentrated under reduced pressure. The requiting residue was subjected to silica gel column chromatography to give 2,83 g of a compound (IX-1).

The ¹H-NMR data of the compound (IX-1) are as follows.
¹H-NMR(CDCl₃, TMS)δ(ppm):3.11-3.18(m, 6H), 3.76(bs, 3H), 4.86(bs, 1.4H), 5.23(bs, 0.6H), 7.17-7.25(m, 1H), 7,57(d, 1H, J=2Hz).

### [Synthesis of Isocyanate Compound (II)]

### <Example 8>

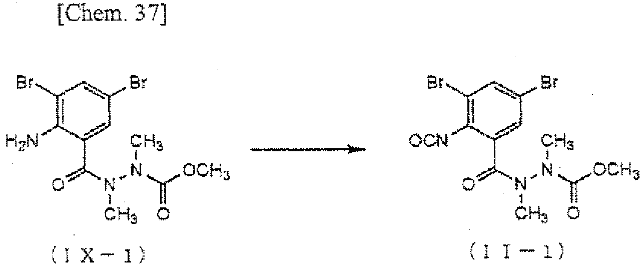

In 2.01 g of toluene was dissolved 0.202 g of the compound (IX-1) and the solution was cooled. Thereafter, a mixed solution of 61.2 mg of triphosgene and 0.52 g of toluene was added dropwise to the solution while being stirred at an internal temperature of 2 to 3°C. The mixture was kept being stirred at the same temperature for 10 minutes and heated to 95°C over 2 hours. The mixture was further heated to 112°C over 2 hours and cooled to prepare an isocyanate compound (II-1)-containing solution.

### [Synthesis of Hydrazine Compound (I)]

### <Example 9>

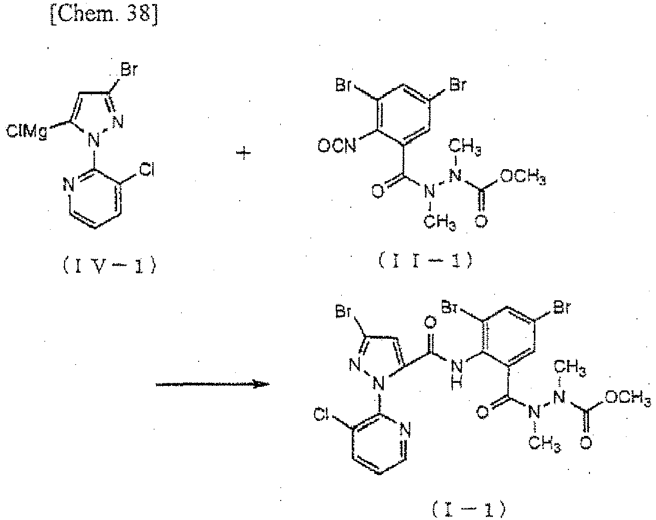

The isocyanate compound (II-1)-containing solution was cooled, and a solution containing the organomagnesium compound (IV-1) obtained in Example 1 was added dropwise thereto over 15 minutes at an internal temperature in the range of 3 to 6°C. Thereafter, cooling was stopped and the mixture was stirred overnight at room temperature. When.the resulting reaction mixture was added with 1.0 g of a saturated ammonium chloride solution at 22.8°C, the temperature rose to 24°C. Then, 1,73 g of water and 1.8 g of ethyl acetate were added to the mixture and the mixture was vigorously stirred. Stirring was stopped. The organic layer, which was taken out by phase-separation, was sequentially washed with 1,7 g of water and 1,7 g of saturated brine and the solvent was removed under reduced pressure to give 0.34 g of a crude product of a hydrazine compound (I-1), Based on HPLC analysis of the crude product, the area percentage of the hydrazine compound (I-1) was 32.3%. The crude product was purified by using silica gel column chromatography to give 96 mg of the hydrazine compound (I-1) (yield: 27.5%).

The ¹H-NMR data of the obtained hydrazine compound (I-1) are as follows. ¹H-NMR(DMSO-d₆, TMS)δ(ppm):2.71(s, 1.4H), 2,83(s, 1,6H), 2.94(s, 1.5H), 3.06(s, 1.5H), 3.35-3.70(m, 3.0H), 7,41(s, 0.5H), 7.45(s, 0.6H), 7.47(s, 0.6H), 7.60-7.64(m, 1,3H), 8.07(d, 0.5H, J=2Hz), 8,13(s, 0,5H), 8.18(d, 1.0H, J=8Hz), 8.50(m, 1.0H), 10.52(s, 0.5H), 10.67(s, 0.5H).

### <Reference Example 4>

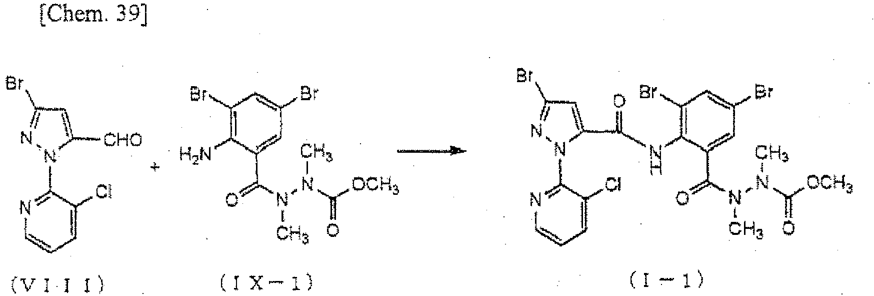

0.33 g of the amino compound (IX-1), 0.24 g of the formyl compound (VIII), 0.25 g of o-chloranif and 2 mL of 1,4-dioxane were mixed, and the mixture was heated with stirring under nitrogen atmosphere for 7 hours under reflux conditions. An aqueous sodium hydrogen carbonate solution was poured into the reaction mixture which was left standing still to cool to room temperature, , and the mixture was extracted with ethyl acetate. The organic layer was washed with water, dried over anhydrous magnesium sulfate, and concentrated under reduced pressure. The resulting residue was subjected to silica gel column chromatography, so that 0.35 g of the hydrazine compound (I-1) was obtained.

The ¹H-NMR. data of the obtained hydrazine compound (I-1) are as follows. ¹H-NNR(DMSO-d₆, TMS)δ(ppm):2.71(s, 1.4H), 2.83(s, 1.6H), 2.94(s, 1.5H), 3.06(s, 1.5H), 3.35-3.70(m, 3.0H). 7.41(s, 0.5H), 7.45(s, 0.6H), 7.47(s, 0.6H), 7.60-7.64(m, 1.3H), 8.07(d, 0,5H, J=2Hz), 8.13(s, 0.5H), 8.18(d, 1.0H, J=8Hz), 8.50(m, 1.0H), 10.52(s, 0,5H), 10,67(s, 0.5H).

### <Reference Formulation Example>

A formulation example when the hydrazine compound (I-1) is used as a harmful arthropod controlling agent is shown in the following.

Ten (10) parts by mass of the hydrazine compound (I-1); 35 parts by mass of white carbon containing 50% by mass of a polyoxyethylene alkyl ether sulfate ammonium salt; and 55 parts by mass of water were mixed and the mixture was pulverized by the wet pulverizing method, so that a 10% flowable was obtained.

The mode of carrying out the invention and Examples disclosed herein should be considered illustrative and non-limited. It is intended that the scope of the present invention is indicated by not the description provided above but the claims and all modification within the meaning of equivalent of claims are included.

## Claims

1. An organomagnesium compound represented by the following general formula (IV): 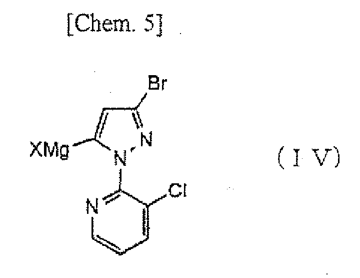 wherein X represents chlorine, bromine or iodine.

2. A method for producing an organomagnesium compound, comprising a step of:
reacting a dibromo compound represented by the following formula (V): 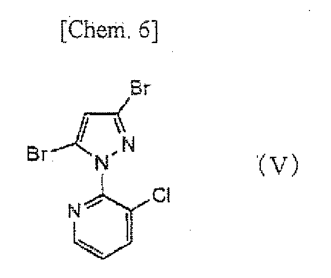
with a Grignard compound represented by the following general formula (VI):
R-MgX (VI)
wherein R represents an alkyl group having I to 6 carbons or a vinyl group; and
X represents chlorine, bromine or iodine,
the organomagnesium compound being represented by the general formula (IV): 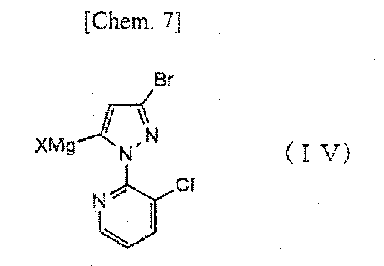 wherein X represents chlorine, bromine or iodine.

3. A dibromo compound represented by the following formula (V):

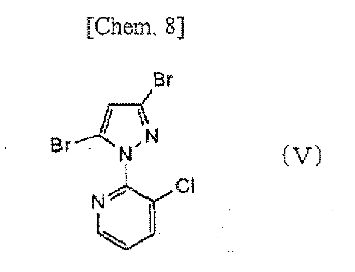

4. A method for producing a formyl compound, comprising a step of:
reacting an organomagnesium compound represented by the following general formula (IV): 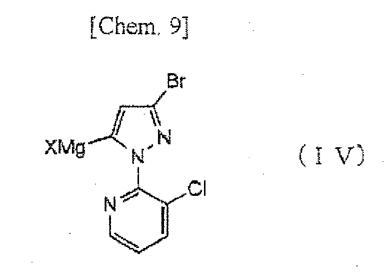 wherein X represents chlorine, bromine or iodine,
with a compound represented by the following genera formula (VII):
HC(=O)-Q (VII)
wherein Q represents an amino group substituted with two alkyl groups each having 1 to 6 carbons or an alkoxy group having 1 to 3 carbons,
the formal compound being represented by the following formula (VIII):
